# EUROPEAN PATENT SPECIFICATION

(11) **EP 3 839 921 B1**
(45) Date of publication and mention of the grant of the patent: **02.07.2025**
(21) Application number: 20215231.0
(22) Date of filing: 17.12.2020
(51) Int. Cl.: G09B 21/00, A61K 9/00, A61K 31/192, A61K 47/10, A61K 47/38

(54) **MULTI-LAYERED BUCCAL DOSAGE FORM AND METHOD FOR THE PRODUCTION THEREOF**
MEHRSCHICHTIGE BUKKALE DOSIERUNGSFORM UND VERFAHREN ZU IHRER HERSTELLUNG
FORME POSOLOGIQUE ORALE MULTICOUCHE ET SON PROCÉDÉ DE FABRICATION

(30) Priority: 17.12.2019 GR 20190100561
(43) Date of publication of application: 23.06.2021
(73) Proprietor: Aristotle University of Thessaloniki - Elke, 54636 Thessaloniki (GR)
(72) Inventor: FATOUROS, Dimitrios, 54 124 Thessaloniki (GR); ELEFTHERIADIS, Georgios, 54453 Thessaloniki (GR)
(74) Representative: Petsis, Christos

(56) References cited:
- US-A1- 2019 270 253
- YU TIAN ET AL: "Oromucosal films: from patient centricity to production by printing techniques", EXPERT OPINION ON DRUG DELIVERY, vol. 16, no. 9, 8 August 2019 (2019-08-08), GB, pages 981 - 993, XP055730678, ISSN: 1742-5247, DOI: 10.1080/17425247.2019.1652595
- ELEFTHERIADIS GEORGIOS K ET AL: "Unidirectional drug release from 3D printed mucoadhesive buccal films using FDM technology: In vitro and ex vivo evaluation", EUROPEAN JOURNAL OF PHARMACEUTICS AND BIOPHARMACEUTICS, ELSEVIER SCIENCE PUBLISHERS B.V., AMSTERDAM, NL, vol. 144, 21 September 2019 (2019-09-21), pages 180 - 192, XP085862481, ISSN: 0939-6411, [retrieved on 20190921], DOI: 10.1016/J.EJPB.2019.09.018
- ANONYMOUS: "Onsolis -FDA prescribing information, side effects and uses WARNING: RISK OF RESPIRATORY DEPRESSION, MEDICATION ERRORS, ABUSE POTENTIAL", 21 November 2019 (2019-11-21), XP055732965, Retrieved from the Internet <URL:https://www.drugs.com/pro/onsolis.html> [retrieved on 20200922]
- H.E. COLLEY ET AL: "Pre-clinical evaluation of novel mucoadhesive bilayer patches for local delivery of clobetasol-17-propionate to the oral mucosa", BIOMATERIALS, vol. 178, 1 September 2018 (2018-09-01), AMSTERDAM, NL, pages 134 - 146, XP055761449, ISSN: 0142-9612, DOI: 10.1016/j.biomaterials.2018.06.009

## Description

### Field of the invention

The present invention relates to a multi-layered buccal dosage form comprising a drug loaded compartment and a backing layer which is drug free and which incorporates a set of identifiers in connection with said load.

### Background of the Invention

Documents US2013/0073068 and US2019/270253 A1 disclose a device, system, respectively a method of solid freeform fabrication of an object, and computer software, wherein the system refers to solid freeform fabrication, an equal term for fused filament fabrication. However, no reference is made in this document to pharmaceutical products. Accordingly, there is no reference to the accuracy of the process to produce specific patterns, which is essential though when it comes to be applied on pharmaceutical products. There is only referred therein to patterns having a thickness of 0,3 mm. According to the International Organization for Standardization and the document ISO/ASTM 52900 (Standard Terminology for Additive Manufacturing - General Principles -Terminology), the accuracy of an additive manufacturing process, such as 3D printing process, is determined as the closeness of agreement between an individual result and an accepted reference value. No data are provided on the accuracy of the printing process, regarding the formation of Braille characters, as long as they describe the Braille characters as candidate patterns.

No specific dimensions of the patterns are provided in said document. As a result, there is no complete reference to the minimum dimensions (x-y-z) of Braille dots that could be efficiently fabricated on the surface of the main object with the specific equipment. However, for pharmaceutical Braille, very specific definitions on the parameters are required to form the encoded text, as described in the European standard EN 15823 and the International standard ISO 17351:2014 (Packaging - Braille on packaging for medicinal products), following the Marburg Medium spacing convention for Braille.

There is no reference to the orientation of the pattern, regarding the position of the main object, or any other process parameters. Consequently, the thickness of the pattern is not correlated to a specific axis during the build cycle. Specific functionalities of the patterns regarding the end-user are not mentioned. Neither are specific functionalities regarding the main object. The design of objects is defined by inputting data regarding their shape, resp. dimensions, and the selection of a suitable material for the achievement of specific physicochemical properties of the object is defined as well, such as mechanical properties, color, magnetic behavior etc.

### Prior art

US201110200537 relates to pharmaceutical products wherein a coated dosage with a backing layer and a method for producing are disclosed. The pharmaceutical dosage forms reported in this document are tablets. This document discloses a coating layer covering the whole body of the object (tablet) and a laser drilling for the formation of identifiers. Laser drilling is a subtractive manufacturing technique. In detail, a coating layer is formed around the tablet, and the laser drilling process selectively removes parts of the layer to result in the distinctive character.

Neither this document nor the related US2019/270253 A1 discloses the formation of identifiers in a multi-step fashion, i.e. production of the tablet by compression -coating of the tablet with a material layer- formation of identifiers by laser drilling. As a result, the identifiers are formed in a post-production process. There are significant differences regarding the Braille dot specifications, as in the case of the latter documents US2013/0073068 and US2019/270253 A1 respectively.

US 5,700,998 discloses a method of coding drug pills. As in said previous documents, the identifiers are formed by a subtractive method, i.e. engraving of the formulation. The formulation is referred as a tablet. No coating or backing layer is disclosed in this document.

The document further discloses the formation of identifiers as a post-production process, regarding the production method of the tablet, a multi-step process. But no reference is made to Braille.

In document CA 2269076, the formation of identifiers is disclosed to be accomplished by compression, local application of heat, embossing, grinding or etching. Again, no reference is made to Braille.

Document US 3,125,490 discloses a tablet with contrasting indicia and method, wherein the pharmaceutical dosage forms are tablets, but not films. Again, the identifiers are formed by compression, and no reference is made to Braille.

Document XP055730678 by Yu Tian details the drug formulation options for special patient groups, e.g. pediatric patients, the elderly with dysphagia, uncooperative or nauseated/vomiting patients. There is no reference to other special groups, as in the case of subjects with visual impairment. The document describes the fabrication of films via two-dimensional inkjet printing, and thus, jetting of a drug-loaded ink onto suitable substrates for inkjet printing. Further, this document reveals the creation of identifying agents on the surface of the films, in the form of quick response (QR) codes, which are intended for the identification of the drug and the critical treatment information by using a special application in mobile phones. This can be rather considered as an asset in the supply chain of pharmaceuticals though, rather than facilitating identification and promoting independence in the identification of pharmaceutical products by visually impaired populations.

Document XP085862481 by Eleftheriadis et al. yet describes the formation of multilayered buccal films by using the 3D printing technology. However, this document has no reference to printing of special identifying structures on the surface of the films, which is presently aimed at with Braille characters for visually impaired patients.

Further, there are commercial buccal films in the drug market, e.g. Onsolis - (https://www.drugs.com/prolonsolis.html). This formulation is marked by a coded identifying number on one side of the film, providing the drug strength. However, this number is only indicative of the dosage strength, and cannot be considered as an identifying agent needed for visually impaired people, which Is the presently targeted population group. This identifying element is debossed on the surface of the films, which is the exact opposite process of embossing, thus producing identifying characters in the form of indents instead of raised characters, whereas only the latter can facilitate identification of the dosage unit by visually impaired patients, and as described in ISO 17351:2014 (Packaging - Braille on packaging for medicinal products). This formulation thus leads away from the target.

Identification of the proper dosage form, that is relevant to a specific active pharmaceutical ingredient or a suitable strength is of critical importance to achieve the optimal therapeutic effects In treatments for diseases. According to the EU Directive 2004/27/EC - Article 56a [1], the incorporation of Braille-encoded information on secondary pharmaceutical packaging (cartons) is mandatory. Since the end of 2010, the name and strength of pharmaceutical products shall be given in Braille format, to facilitate identification of the medicinal products intended for human use by visually impaired populations.

Toward this direction, the cooperation between the European Blind Union (EBU) and the Royal National Institute of Blind people yielded the unified specifications for Braille labeling of pharmaceutical products. Special attention was given to the dot height developed on the pharmaceutical packaging via embossing, to optimize the large variances achieved by the current Braille-generating technology, as well as the deviations presented among the countries in the European Union ^{[2,3]}.

The conducted research on the specifications of the dots resulted in the establishment of the Marburg Medium spacing convention for Braille, exclusively intended for industrial use in pharmaceutical packaging. This standard is recommended and described by the European Committee for Standardization (EN 15823) ^{[4]} and the International Organization for Standardization (ISO 17351:2014) ^{[5]}.

However, a variety of pharmaceutical products exist in the market, containing individually enclosed dosage forms with varying scales of drug dose, e.g. Emend^{®} (Aprepitant, 125 mg/80 mg) or intended for multiphasic self-administration during the treatment period provided to the patient, e.g. Divina (Estradiol Valerate 2 mg / Estradiol Valerate 2 mg and Medroxyprogesterone Acetate 10 mg). In such cases, Braille encoding in the external packaging is considered problematic, due to inconvenient identification -or at least unsatisfactory- of the proper strength by visually impaired people, which thus again leads away from the target.

To summarize, 3D Braille printing as such is known in the art yet.

The 3D printing of pharmaceutical multilayer films for buccal administration is known yet from XP055730678 and XP085862481, but not 3D printing of Braille characters on medicinal products. In the prior art, no Braille-type medicinal product (oral mucosa, pills) was detected on the drug. Even more, there is no disclosure in the prior art of Braille 3D printing on the back layer of a multilayer oral mucosa.

Embossing of characters on pills is also generally known in the art.

XP055730678 yet discloses a buccal dosage form intended for buccal administration of a pharmaceutically active substance to users, which is multilayer yet as bilayer and multilayer films, and comprises a first layer consisting of a drug-loaden film compartment and backing layer as a second layer forming a backing layer to which said first layer is attached, wherein it further comprises a set of identifiers arranged in said second layer of said form.

XP085862481 also discloses a buccal dosage form for buccal administration of a pharmaceutically active substance which is multilayered and comprises a first layer consisting of a film compartment loaded with a drug and backing layer as a second layer forming a backing layer to which said first layer is attached with the development of 3D printed buccal films, also with a set of identifiers arranged in said second layer of said form.

Other known problems are related to the dimensions of the dots, maintaining their target specifications over time. Post-production alterations on the initially set target are often evidenced. This phenomenon, widely known as "settling", takes place over a large period after initial production, related to the nature of the material, the original dot height, as well as storage, transportation, and handling conditions ^{[2]}.

From another point of view, there are several therapies for diseases that require varying dosage of the active ingredient, although the pharmaceutical products are available in the market in quantized strengths. In such cases, management and/or segmentation of the marketed product is a common approach even from health professionals, to achieve the required dose strengths with unknown bioavailability and unwanted pharmacokinetic behaviour.

According to prior art, the formation of various patterns on the surface of flat objects, but non-related to pharmaceutical products, has been reported via solid freeform fabrication ^{[6]}. Furthermore, different kinds of encoded identifiers onto pharmaceutical products have been realized by various methods. But none of them reached the aimed achievement to be fulfilled according to the presently targeted development. Principally, the complete process included multiple steps for the preparation of the final product. The identifiers were generated by compression ^{[7,8]}, engraving ^{[9]}, laser drilling ^{[10]}, local application of heat ^{[8]}, embossing ^{[8]}, grinding or etching ^{[8]},

### Aim of the invention

The invention aims at providing a solution to the technical problem stated above. The present invention thus aims to the simultaneous resolution of the highlighted complications related to the development of Braille-characters on the secondary pharmaceutical packaging of personalized medications and to allow a haptic perception thereof by the targeted visually impaired populations. The present invention is considered an industrially oriented approach, in the context of patient-centricity, with special attention to visually impaired populations.

### Summary of the Invention

The invention relates to a multi-layered buccal dosage form, as defined in claim 1 as a buccal dosage form intended for the buccal administration of an active pharmaceutical compounds formulation to visually impaired users and a production method and apparatus therefore as defined in the other independent claims.

According to a further particular embodiment of the buccal dosage form of the invention, the identifiers are arranged in a rectangular pattern with varying dimensions with proper dimensions of said rectangle patterns, by means whereof dosage forms have different doses of the active pharmaceutical ingredient API.

According to a more particular embodiment of the buccal dosage form according to the invention, the area of the Braille-containing compartment of said backing layer is in compliance with the dimensions of the drug-loaded compartment of said first layer.

According to a yet more particular embodiment of the buccal dosage form according to the invention, said second layer forming a screen limits the drug release towards the oral cavity of the a visually impaired user, by protecting the drug-loaded compartment of said first layer from the salivary fluids of said user, thus preventing an excessive swallowing of the released drug by said user, wherein the second layer enhances an unidirectional release of the drug toward the buccal epithelium of the user, particularly wherein the thickness of said backing layer is about 0,1 mm.

A specific embodiment of the buccal dosage form of the invention is as defined in claims 6, respectively 7.

The present invention also relates to a method for producing a multi-layered buccal dosage form, wherein the invention's method is as defined in claim 8.

Accordingly, the proposed invention includes a method of fabricating drug-loaded films attached to a backing layer, which incorporates Braille-encoded characters, to facilitate the haptic identification of the correct dose of the active pharmaceutical compound by visually impaired populations, which is to be considered a very specific and narrow field. The dosage forms are intended for the buccal administration of active pharmaceutical compounds. The presence of a backing layer actually forms a screen acting as a limiting barrier against the release of the drug toward the oral cavity, which thus prevents the extended swallowing of the released drug and promotes instead a unidirectional release toward the buccal epithelium, as it suits. Furthermore, the backing layer provides the substrate for the formation of the Braille characters thereon, so as to protrude therefrom.

The current invention details the development of a multi-layered buccal dosage form by said FFF. The formulation comprises a drug-loaded compartment, presenting different dosage strengths of the active pharmaceutical compound or ingredient designated hereafter as API and a drug-free compartment, providing the properties of a backing layer. During the same of the build cycle of the said FFF process, Braille characters are formed onto the backing layer, so as to protrude therefrom indicating the dosage strength of the formulation, in order to make them palpable by a visually impaired user, on the one hand, and also perceptible by the latter with regard to the said dosage strength, which is a critical issue here.

In the present invention, the patterns are oriented toward the z-axis, regarding the orientation of the surface of the drug-loaded compartment. The global orientation of z-axis is defined in ISO/ASTM 52900.

Thus, the object according to the invention notably differs from the above depicted closest state of the art in the characteristics associated with the identifiers which form Braille characters on the backing layer and which convey information about the particular drug load of the film. Besides, US2019/270253 A1 does not involve drug-layered laminates. 3D Braille printing as such is also known therein. However, there is no evidence in the prior art of 3D printing on the defined pharmaceutical films, and therefore, there is no evidence of 3D printed Braille characters on medicinal products.

Although references to 3D printing are essentially only in the method of production using 3D printing, Braille characters on medicinal products before were not disclosed in said prior art. So, there is no relevant disclosure of the distinctive features referred to above. As commercially available films, such as e.g. Onsolis in XP055732965 are known yet to carry dose power printed on them in visible characters for the sighted in "Dosage Forms and Power" which is per se not applicable though for visually impaired users, precisely because they cannot see, even though Braille characters may be placed directly on the film.

According to a more particular embodiment of the method according to the invention, the invention is as defined in claim 10.

According to a still more particular embodiment of the method of the invention, drug-free filaments and drug-loaded filaments of hydroxypropyl methylcellulose, HPMC, are produced using said Hot Melt Extrusion process step a, wherein the first phase consists of said Hot Melt Extrusion of filaments and with specific parameters, preparing said filaments, wherein said filaments constitute the feedstock or raw materials for the realization of said FFF process step b, on the one hand, and a subsequent phase defining the fabrication procedure in the prescribed order of steps, consisting of the design of buccal films with Braille characters, on the other hand.

A preferred embodiment according to the invention thus includes an example wherein the production process comprises at least the following phases: drug-free and drug-loaded filaments of hydroxypropyl methylcellulose HPMC, drug-free and drug-loaded filaments resp. are produced using a hot melt extrusion process. The hot melt extrusion process of said drug-free filaments and drug-loaded filaments is detailed hereafter, which process comprises at least the following phases a & b: first an a phase including a Hot Melt Extrusion of filaments and specific parameters. This phase a represents the preparation of filaments. Filaments are the feedstock (raw materials) for the realization of the FFF process b. In a similar manner, the design-procedure of the objects which is the way that the shapes are designed in the CAD software, will be a separate phase from the fabrication of the formulations.

According to a preferred embodiment of the method of the invention, drug-loaded filaments and drug-free filaments of HPMC are produced using
- said hot melt extrusion process a, consisting of a Hot Melt Extrusion with specific parameters that represent the preparation of said filaments, which filaments are the feedstock or raw materials for the realization of the FFF process, and
- a phase b for the fabrication procedure itself, as such, consisting of the design of buccal films with Braille characters.

According to a specific embodiment of the method of the invention, the filaments of HPMC are produced with different compositions using a single-screw hot melt extruder, which is operated at a certain screw speed, particularly of substantially 35 rpm, wherein the composition of filament is of substantially 92% w/w HPMC and 8% w/w Ketoprofen, and the composition of filament is of substantially 95% w/w HPMC and 5% w/w polyethylene glycol PEG 400, wherein the extrusion process for both filaments is conducted in the range of 172-174°C, herewith increasing the flow of the molten polymer.

Said filaments A' and B' of HPMC with different compositions are produced using a single-screw hot melt extruder (Filabot Original, Filabot Inc., VT, USA). The device operates at a screw speed of 35 rpm. The composition of filament A' was 92% w/w HPMC and 8% w/w Ketoprofen. The composition of filament B' was 95% w/w HPMC and 5% w/w polyethylene glycol PEG 400. The extrusion process for both filaments was conducted in the range 172-174°C, to improve the flow of the molten polymer in the heating-chamber of the device. The device was equipped with a nozzle of 1,6 mm diameter, to result in filaments with a target diameter of 1,75 ± 0,05 mm, taking into consideration the Barus effect.

The drug loaded compartment of said first layer and the backing layer containing the Braille characters, resp. the rectangle patterns with varying dimensions can be designed in a computer-aided script-based design software (OpenSCAD), considering the Ketoprofen content of the drug-loaded filaments, wherein the dimensions of the rectangle patterns are selected to produce dosage forms with different doses of the active pharmaceutical ingredient API, wherein the drug-loaded compartment is prone to drug release toward all directions, particularly wherein the whole design containing said drug-loaded compartment, said backing layer, and said Braille dots, is exported as a stereolithography (.stl) file format.

According to another embodiment of the method of the invention, the dosage forms are films, wherein the formation of the drug-loaded compartment, the backing layer and the identifiers are solely fabricated in a one-step process expressed as one "build cycle", by 3D printing.

The present invention further also relates to a device for carrying out a method as defined above wherein said device comprises a single-screw hot melt extruder, operating at a certain screw speed, particularly of substantially 35 rpm, by means whereof both filaments A' and B' of HPMC are produced with different compositions, particularly wherein the composition of the one filament is of substantially 92% w/w HPMC and 8% w/w Ketoprofen, and the composition of the other filament is of substantially 95% w/w HPMC and 5% w/w polyethylene glycol, PEG 400, wherein the extrusion process for both filaments is conducted in the range of 172-174°C, herewith increasing the flow of the molten polymer in the heating-chamber of said device, wherein said device is equipped with a nozzle of a diameter particularly of substantially 1,6 mm, thereby yielding filaments with a target diameter of 1,75 ± 0,05 mm.

Finally, the present invention also relates to a method as defined in claim 14, wherein the produced filaments are utilized for the fabrication of buccal films with Braille characters in a three-dimensional 3D printing process, to manufacture the dosage forms, based on the designed digital stl templates, wherein the FFF process is performed using a specified printer at an extrusion temperature of 200°C, to generate a smooth flow of the molten polymeric filament from the nozzle of the device, and a build platform temperature of 90°C, thereby increasing adhesion of the object on the platform during the build cycle, wherein the printing speed is set to 30 mm/s, by means whereof the Braille dots are accurately reproduced onto the free surface of the backing layer, wherein the process parameters are properly selected with consideration of the specific material used.

In the invention, the Braille patterns characterize the object as It is, by indicating specific properties of the drug loaded compartment, e.g. drug dose, directly to the visually impaired user in that the drug dose is transformed to specific dimensions of the object, to achieve the target strength of said API in such a way that this essential information is directly by the interested user who is visually impaired, only by a simple tactile contact between this user and the protruding identifiers, to allow them to get right away the medical and pharmaceutical information he needs for taking the right medication with the good dosage. Thus, there is a significant difference in the inputting data and the target property of the fabricated object, compared to prior art.

### Brief description of drawings

Figure 1 represents a separate design of a drug-loaded compartment, a backing layer and the Braille characters in exploded view, related to formulation F0 with a certain dose strength according to the invention.
Figure 2 represents a preferred embodiment of a final multi-layered buccal dosage form according to the Invention corresponding to figure 1.
Figure 3 is a representative digital stereoscope image of Braille dots formed onto a buccal formulation F0 with the latter said dosage strength, in compliance with the Marburg Medium specifications.
Figure 4 is a similar representative digital stereoscope image of Braille dots formed onto a buccal formulation F2 with a dosage strength lower than in figure 3, with dimensions and spacing significantly enhanced, compared to the Marburg Medium standard.

### Description

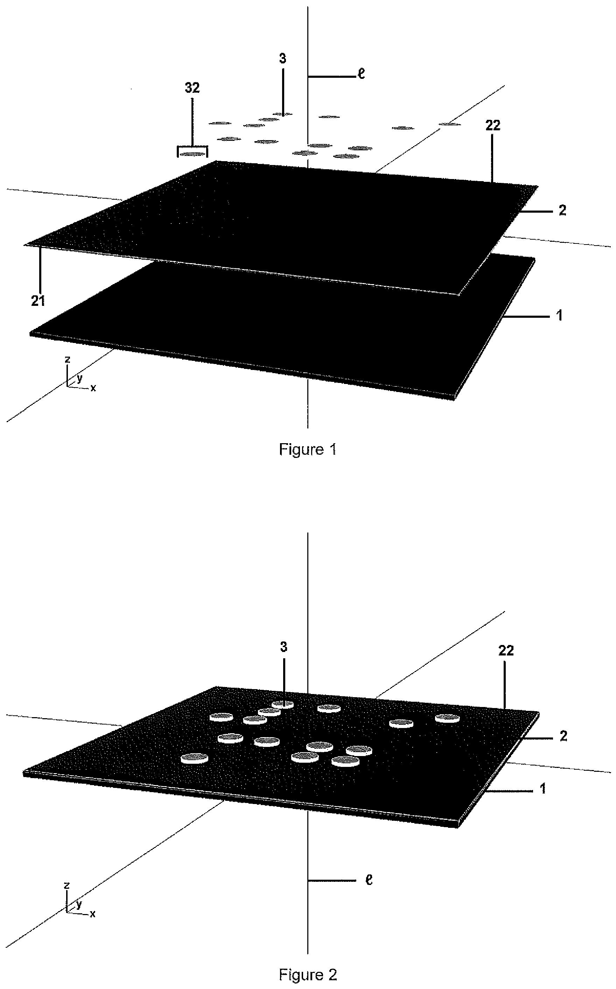

Figure 2 shows the invention relating to a multi-layered buccal dosage form 10, which comprises a drug loaded compartment 1 and a backing layer 2, incorporating Braille characters 3 protruding therefrom, wherein Figure 2 represents a preferred embodiment of a final multi-layered buccal dosage form F0, with a dose strength of 19 mg. The invention also relates to a method for producing the latter, wherein the production process may comprise two phases: a first phase **a** in connection with the development of feedstock by hot melt extrusion and a second phase **b** in connection with 3D printing of buccal films with braille characters by FFF as shown in Figure 1.

The drug loaded compartment 1 and the backing layer 2, containing the Braille characters 3, are designed in a computer-aided script-based design software (OpenSCAD). The description of the design of buccal films is detailed hereafter: after the abovementioned phase **a,** a subsequent phase **b is** carried out which is indicative of the fabrication procedure, wherein an overview of the exact sequence of steps followed is set out.

The design of buccal films 10 with Braille characters 3 is performed. Rectangle patterns 31 with varying dimensions were designed in a computer-aided script-based design software (OpenSCAD). Considering the Ketoprofen content of the drug-loaded filaments 1, proper dimensions of the rectangle patterns 31 were selected, to facilitate the development of dosage forms 10 with different doses of the API shown in Figure 1 and represented in Table 1 below. The drug-loaded compartment 1 is prone to drug release toward all possible directions.

**Table 1**

| Sample | Strength | Diameter | b1 | b2 | h1 | h2 |
|---|---|---|---|---|---|---|
| | [mg] | [mm] | [mm] | [mm] | [mm] | [mm] |
| F0 | | 1,58 ± 0,06 | 2,52 ± 0,06 | 6,03 ± 0,05 | 10,03 ± 0,05 | 2,51 ± 0,06 |
| | 76 | 1.59 ± 0.05 | 2,53 ± 0,06 | 6,01 ± 0,06 | 09,99 ± 0,06 | 2,52 ± 0,06 |
| F1 | 19 | 1,57 ± 0,05 | 2,52 ± 0,06 | 6,02 ± 0,06 | 10,00 ± 0,05 | 2,53 ± 0,05 |
| | 76 | 1.58 ± 0,05 | 2,52 ± 0,07 | 6,03 ± 0,06 | 10,02 ± 0,04 | 2,51 ± 0,06 |
| F2 | | 2,04 ± 0,06 | 3,12 ± 0,07 | 7,51 ± 0,07 | 12,13 ± 0,06 | 3,13 ± 0,05 |
| | 76 | 2,03 ± 0,06 | 3,13 ± 0,06 | 7,52 ± 0,07 | 12,09 ± 0,07 | 3.13 ± 0,06 |

Table 1 shows the determination of the dimensions of the 3D-printed Braille dots, as described in ISO 17351:2014 (mean ± SD, number of samples = 20).

The area of the Braille-containing compartment 2 (in x-y orientation) is designed in compliance with the dimensions of the drug-loaded compartment 1, whereas the thickness is 0,1 mm. The backing layer 2 is intended to act as a substrate for the formation of Braille identifiers 3, and as an isolating screen serving as effective barrier which limits the drug release towards the oral cavity, by protecting the drug-loaded compartment 1 from the salivary fluids of the targeted user. Thus, the incorporation of the backing layer facilitates the release of the drug toward the buccal epithelium of the visually impaired user.

The Braille characters 3 are designed on top 22 of the backing layer 2. In one embodiment, the Braille characters 3 were designed in compliance with the Marburg Medium spacing convention for Braille, recommended by the European Blind Union (EBU) for pharmaceutical packaging, i.e. 1,6 mm dot diameter 32, 2,5 mm horizontal and vertical dot spacing 35 and 36 respectively, 6,0 mm horizontal character spacing 10,0 mm vertical line spacing (h1). The dot height 37 was set at 0,2 mm. For comparison purposes modifications over the EBU specifications have been further evaluated. Thus, in another embodiment, the dot height 37 was raised at 0,3 mm. In a further embodiment, the Braille characters were formed by setting the dot diameter 32 to 2,0 mm, the horizontal and vertical dot spacing 35,36 resp. to 3,1 mm, the horizontal character spacing to 7,5 mm and the vertical line spacing to 12,0 mm. The dot height 37 was set at 0,4 mm.

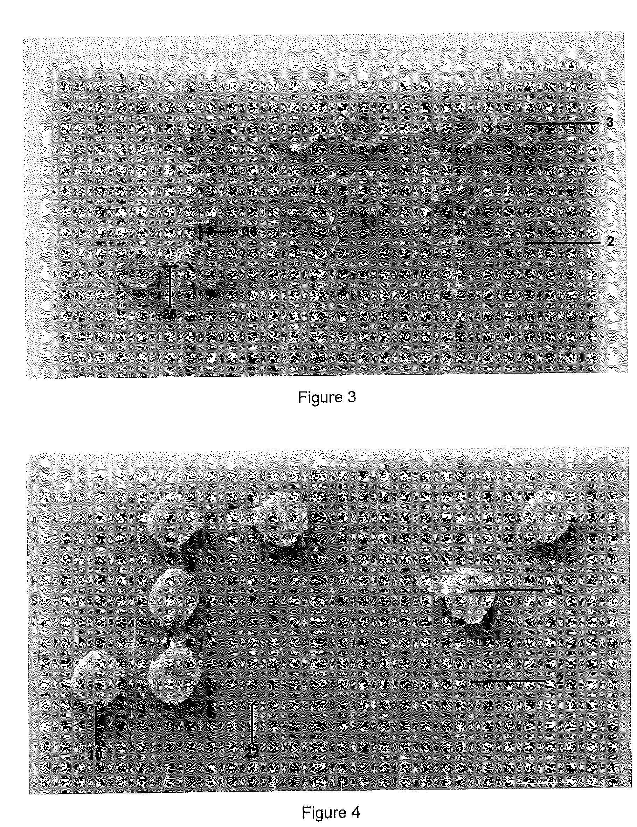

The Braille-containing backing-layer 2 was subsequently attached to the top surface of the drug-loaded compartment 1. The whole design shown in Figure 3, containing the drug-loaded compartment 1, the backing layer 2, and the Braille dots 3, was exported as a stereolithography stl file format, wherein Figure 3 is a representative digital stereoscope image of Braille dots formed onto a buccal formulation F0 with a dosage strength of 76 mg, in compliance with the Marburg Medium specifications.

Fabrication of buccal films with Braille characters 3 is further set out. The produced filaments were utilized in a three-dimensional 3D printing process, i.e. FFF, to manufacture the dosage forms, based on the designed digital stl templates. The FFF process was performed using a Makerbot Replicator 2X printer (Makerbot Inc., USA), at an extrusion temperature of 200°C, to facilitate the smooth flow of the molten polymeric filament from the nozzle of the device, and a build platform temperature of 90°C, to enhance the adhesion of the object on the platform during the build cycle. The printing speed was set to 30 mm/s, to accurately reproduce the developed Braille dots 3 onto the surface of the backing layer 2. The process parameters were properly selected, with consideration of the specific material used.

In one embodiment, the dimensions of the drug-loaded compartment 1 are selected to produce a dose strength of 19 mg. The rationale behind the selection of the specific dose strength was to result in a Braille format, with the most sparse distribution of Braille dots 3, scattered over the area of the backing layer 2, to evaluate the accuracy and repeatability of the 3D printing process, as well as the readability of the Braille characters 3 by visually impaired people.

In another embodiment, the dimensions of the drug-loaded compartment 1 are selected to result in a dose strength of 76 mg. The rationale behind the selection of the specific dose strength was to result in a Braille format with more dense distribution of Braille dots over the area 3 of the backing layer 2 (compared to 16 mg), to evaluate the accuracy and repeatability of the 3D printing process, as well as the readability of the Braille characters 3 by visually impaired people.

In a further embodiment, the Braille characters are designed in compliance with the Marburg Medium spacing convention for Braille, i.e. 1,6 mm dot diameter 32, 2,5 mm horizontal and vertical dot spacing 35,36 resp., 6,0 mm horizontal character spacing 10,0 mm vertical line spacing. The dot height 37 was set at 0,2 mm (formulations F0).

In another embodiment, the specifications of the Braille dots 3 were modified, for comparison reasons, i.e. 1,6 mm dot diameter, 2,5 mm horizontal and vertical dot spacing, 6,0 mm horizontal character spacing, 10,0 mm vertical line spacing. The dot height 37 was set at 0,3 mm (formulations F1).

In a further embodiment, the specifications of the Braille dots 3 were modified, for comparison reasons, i.e. 2,0 mm dot diameter, 3,1 mm horizontal and vertical dot spacing, 7,5 mm horizontal character spacing, 12,0 mm vertical line spacing. The dot height 37 was set at 0,4 mm (formulations F2).

The filaments A' and B' are loaded in the FFF printer, as feedstock for the fabrication of the drug-loaded compartment 1 and the backing layer 2 containing the Braille characters 3, respectively. The fabrication process of buccal films and the selected parameters is detailed above. The FFF process parameters are selected to accurately reproduce the final formulations, considering a tolerance of ± 0,1 mm for the dimensions of the produced Braille dots, as proposed in the International Standard ISO 17351:2014. This represents the contribution of the specific parameters to the technical effect.

Results related to the morphological assessment of the printed Braille dots are set out hereafter with morphology of the 3D printed Braille dots. The potential of 3D printing to develop Braille characters 3 over polymeric buccal films, complying with the EBU recommendation, was assessed by a Digital stereoscope (Celestron Digital Microscope Pro, Celestron, California, USA). The obtained images shown in Figures 3-4 presented excellent repeatability in the production of Braille dots 3 as visible from abovementioned Tables 1 & 2 below, wherein Figure 4 is a similar representative digital stereoscope image of Braille dots formed onto a buccal formulation F2 with a dosage strength lower than in Figure 3, with dimensions and spacing significantly enhanced, compared to the Marburg Medium standard, with a dosage strength of 19 mg.

**Table 2**

| Sample | Strength [mg] | Pre-evaluation dot height [mm] | Post-evaluation dot height [mm] |
|---|---|---|---|
| F0 | 19 | 0,20 ± 0,03 | 0,21 ± 0,02 |
| | 76 | 0,19 ± 0,02 | 0,19 ± 0,03 |
| F1 | 19 | 0,29 ± 0,02 | 0,30 ± 0,02 |
| | 76 | 0,29 ± 0,03 | 0,29 ± 0,02 |
| F2 | 19 | 0,41 ± 0,02 | 0,40 ± 0,03 |
| | 76 | 0,41 ± 0,02 | 0,40 ± 0,02 |

Table 2 shows the determination of the height of the 3D-printed Braille dots, before and after haptic evaluation by visually impaired people (mean ± SD, number of samples n = 20).

The dimensions and spacing of dots 3 for formulations F0 (x-y plane) were in agreement with the EBU specifications for pharmaceutical packaging and labeling, considering a tolerance of ± 0,1 mm for the dimensions of the produced Braille dots 3 (ISO 17351:2014). The dot height 37 was verified in at least three places over the area of printed text by covering at least three Braille dots 3, using a spring-loaded micrometer with a spring-force of 5 N (ISO 17351:2014). The recorded values for formulations F0 satisfied the criteria for embossed materials or other production methods (ISO 17351:2014). In all cases, the dot height 37 was in good agreement with the predesigned patterns. The results were indicative of the accuracy of the FFF process toward the development of buccal dosage forms 10 containing Braille-encoding, which complies with the EBU recommendations.

An in vivo Assessment of the 3D-printed Braille text is presented hereafter.

The fabricated formulations were subjected to haptic assessment by visually impaired people. The protocol for conducting this study was approved by the Research Ethics Committee of Aristotle University of Thessaloniki (28625/2019 and 89262/2019). The participants (N = 15) were recruited with the contribution of the Center for Education and Rehabilitation for the Blind (CERB, Thessaloniki, Greece).

The participants were asked to provide details on their Braille experience, expressed in years, and the frequency of using their Braille-reading skills. The categorization of the characteristics of the participants are summarized in Table 3 below wherein it represents main characteristics of the participants with N = 15.

**Table 3**

| Characteristic | N |
|---|---|
| Braille experience | |
| 2-25 years | 9 |
| 25-50 years | 6 |

| Reading frequency | |
|---|---|
| Daily | 7 |
| Often | 5 |
| Rare | 3 |
| Total | 15 |

Table 3 shows the haptic evaluation of the 3D printed formulations by visually impaired people.

The participants were asked on their opinion on the current state of Braille encoding on pharmaceutical packaging. The participants described the identification of Braille information as problematic, related to a variety of issues, as presented in Table 4 below showing the reported issues identified on the current state of Braille on pharmaceutical packaging with N = 15:

**Table 4**

| Issue | N |
|---|---|
| Material quality | 10 |
| Handling related deformation | 10 |
| Braille quality | 8 |
| Time related deformation | 4 |
| Label covering | 2 |

In most of the cases, the poor material quality and the handling-related deformation of the Braille sets were the main reported issues.

The participants were asked to evaluate the presence of Braille text onto the formulations of the study. The reported scores are summarized in Table 5 below.

**Table 5**

| Sample | Strength | Score | Total score |
|---|---|---|---|
| | [mg] | [mean ± SD] | [mean ± SD] |
| F0 | 19 | 4,33 ± 0,70 | 4,37 ± 0,66 |
| | 76 | 4,40 ± 0,61 | |
| F1 | 19 | 4,40 ± 0,61 | 4,40 ± 0,61 |
| | 76 | 4,40 ± 0,61 | |
| F2 | 19 | 3,67 ± 0,90 | 3,60 ± 0,95 |
| | 76 | 3,53 ± 0,90 | |

Table 5 shows the haptic evaluation of the 3D printed formulations by visually impaired people.

The similar performance of formulations F0 and F1 was evidenced, presenting scores of 4,37 ± 0,66 and 4,40 ± 0,61, respectively, indicative of the insignificant effect of increasing the dot height 37 from 0,2 mm (F0) to 0,3 mm (F1). The minimum SD values represented the optimal convergence of the recorded scores, highlighting the insignificant effect of experience and reading habits of the participants on the identification and the specifications preference of Braille sets.

Modifications over the Marburg Medium spacing convention for Braille rendered a negative haptic performance, as indicated by the evaluation of F2 with a score of 3,60 ± 0,95. Moreover, the maximum SD value indicated the divergent view of the participants, regarding their background on Braille reading and experience, as well as their personal preference.

After identification and evaluation of the Braille sets, the participants were asked to further examine the formulation for at least 5 min. The aim behind this request was to evaluate the effect of extended human handling on the dot height. The post-evaluation values of the redetermined dot heights 37 is presented in Table 2 above. It was evidenced that the Braille sets maintained their initial dot height.

The overall impression of the participants on the fabrication of Braille encoding over the body of the dosage form was recorded. The participants identified several cases in which the reported invention stands superior to the current state of Braille texts in pharmaceutical packaging as represented in Table 6 below. In terms of user independence, the reported invention provides the ability of massive storage of dosage forms by visually impaired people, as in the case of loss or deformation of the original packaging, as well as for multiphasic administration and transferring issues.

**Table 6**

| Issue | N |
|---|---|
| Loss of packaging | 8 |
| Deformation of packaging | 7 |
| Multiphasic administration | 7 |
| Easier transfer | 6 |
| Better protection | 4 |
| Personalized dosage | 4 |

Table 6 shows cases in which the participants identified the reported invention as superior to the current state of pharmaceutical Braille.

### References

[1] Directive 2004/27/EC of the European Parliament and the Council on the Community code relating to medicinal products for human use (2004) Official Journal of the European Union L136, p. 48.
[2] Douglas, G. Weston, A., Whittaker, J., Morley Wilkins, S. and Robinson, D. (2008) Final Report: Braille dot height research: Investigation of Braille Dot Elevation on Pharmaceutical Products (University of Birmingham) (ISBN: 0704426919/9780704426917)
[3] Douglas G., Weston, A., Whittaker, J., Morley Wilkins, S. and Robinson, D. (2009) An Investigation of the Height of Embossed Braille Dots for Labels on Pharmaceutical Products. Journal of Visual Impairment & Blindness, 103, 10, 662-667
[4] European Standard EN 15823:2010 "Packaging - Braille on packaging for medicinal products" (2010)
[5] International Standard ISO 17351:2014 "Packaging - Braille on packaging for medicinal products" (2014)
[6] US Patent 2013/0073068, Napadensky, Mar. 21, 2013
[7] US Patent 3,125,490, Hershberg, Mar. 17, 1964
[8] Canadian Patent 2,269,076, Apr. 16, 1999
[9] US Patent 5,700,998, Palti, Dec. 23, 1997
[10] US Patent 2011/0200537, Szymczak, Aug. 18, 2011

These documents are further described and referenced in the present invention.

## Claims

1. Buccal dosage form intended for the oral administration of an active pharmaceutical compounds formulation to visually impaired users, which is multi-layered,
wherein it comprises a first layer (1) consisting of a drug loaded film compartment and a second layer (2) to act as a substrate for the formation and as an isolating screen serving as effective barrier which limits the drug release towards the oral cavity or promoting unidirectional release of the drug and forming a support layer for formation of Braille identifiers (3), on which said first layer (1) is arranged and attached thereto at a first side (21) of said second layer (2),
wherein said second layer (2) further comprises a set of dedicated identifiers (3) which are arranged on said second layer (2) of said dosage form (10) representing the load,
wherein said second layer (2) constitutes a substrate for said identifiers (3) in said dosage form (10) on the side (22) thereof (2) which is opposite to its said fixation side (21),
wherein said Identifiers (3) indicate the dosage strength of said formulation, wherein said identifiers (3) are grouped together into predetermined identifiers patterns (31) corresponding to a specific load,
in that said identifiers (3) are perceptible by said visually impaired user straightforwardly by a tactile contact therewith, and
in that said identifiers (3) are formed by Braille-coded characters which are supported on said second layer (2) and which are protruding from said second layer (2), thereby forming specific Braille-coded patterns (31), wherein each said specific Braille-coded pattern (31) represents a specific load individually for said visually impaired user.

2. Buccal dosage form according to claim 1, **characterized in that** said first layer (1) with said drug-loaded compartment comprises a variety of dosage strengths of said active pharmaceutical compound API, where API stands for active pharmaceutical ingredient, and **in that** said second layer (2) is drug-free, thereby respectively constituting an active layer and a passive support layer, wherein said dosage form thus includes an active layer and a passive layer with a mutually different but complementary functionality.

3. Buccal dosage form according to claim 1 or 2, **characterized in that** said predetermined identifiers pattern (31) is rectangular with varying dimensions, by means whereof dosage forms have different doses of said active pharmaceutical ingredient API.

4. Buccal dosage form according to any one of the claims 1 to 3, **characterized in that** the area of said identifiers-supporting compartment of said second layer (2) is in compliance with the dimensions of the drug-loaded compartment of said first layer (1).

5. Buccal dosage form according to any one of the claims 1 to 4, **characterized in that** said isolating screen limits the drug release towards the oral cavity of a visually impaired user, by protecting the drug-loaded compartment of said first layer (1) from salivary fluids of said user, thus preventing an excessive swallowing of the released drug by said user, wherein the second layer (2) enhances an unidirectional release of the drug toward the buccal epithelium of said visually impaired user, particularly wherein the thickness of said second layer is 0,1 mm.

6. Buccal dosage form according to any one of the claims 1 to 5, **characterized in that** said identifiers (3) have a substantially cylindrical shape protruding substantially orthogonally from the free surface (22) of the second layer (2) as Braille characters (3) located thereon extending axially along respective vertical axis (I) mutually parallel.

7. Bucal dosage form according to any one of the claims 1 to 6 **characterized in that** said Braille characters have a dot diameter (32) of substantially 1,6 mm, a horizontal and vertical dot spacing (35), resp. (36) of substantially 2,5 mm, a horizontal character spacing of substantially 6,0 mm, a vertical line spacing of substantially 10,0 mm, wherein the dot height (37) is substantially 0,2 mm.

8. Method for producing a multi-layered buccal dosage form as defined in any one of the claims 1 to 7, **characterized in that** said identifiers (3) are formed in accordance with the load according to a predetermined functional scheme, particularly based on a proportionality relationship therebetween identifier (3) versus load,
wherein said drug-loaded film (1) is formed and it (1) is attached to said second layer (2), wherein said identifiers (3) are formed as Braille-encoded characters allowing a tactile identification of a target dose of the active pharmaceutical compound by a visually impaired user and wherein the Braille format is obtained from the target dose strength of the active compound.

9. Method according to claim 8, **characterized in that** it comprises at least two phases including
- a first step **a** consisting of generating feedstock by hot melt extrusion and
- a second step **b** consisting of forming a buccal film as second layer (2) with building Braille characters (3) thereon (2) by 3D printing by fused filament fabrication FFF,
in particular thus providing an integrated structure.

10. Method according to the preceding claim 9, **characterized in that** the formulation is generated from a fixed combination of said drug-loaded compartment as active first layer (1) presenting different dosage strengths of the required active pharmaceutical compound API with superposition on said drug-free compartment providing the properties of said backing layer (2), wherein during the same single phase of the building cycle of said FFF process, said Braille characters (3) are formed onto the backing layer (2), wherein said Braille characters (3) indicate the dosage strength of the formulation straightforwardly to the user by simple tactile contact, **in that** they (3) are further grouped together into specific Braille patterns (31), which characterize the object by indicating specific properties of said drug loaded compartment of said first layer (1), such as drug dose, wherein said drug dose is transformed into specific dimensions of said object so as to achieve the target strength of the active compound, for which an additional manufacturing process is performed wherein a layer-upon-layer material deposition is made onto the backing layer (2) resulting in that the identifiers (3) are formed by said layer-upon-layer material deposition onto the backing layer (2), for a haptic apprehension of the required information contained therein (3) by the relevant user who is visually impaired.

11. Method according to claim 9 or 10, **characterized in that** drug-free filaments **B'** and drug-loaded filaments **A'** of hydroxypropyl methylcellulose HPMC, are produced by carrying out the following steps in the prescribed order:
- said Hot Melt Extrusion process step **a,** wherein the first phase consists of said Hot Melt Extrusion of filaments with specific parameters preparing said filaments, wherein said filaments constitute the feedstock or raw materials for the realization of said FFF process step **b,** and
- a subsequent phase defining the fabrication procedure, consisting of the design of buccal films with Braille characters (3).

12. Method according to claim 11, **characterized in that** the filaments **A'** and **B'** of HPMC are produced with different compositions using a single-screw hot melt extrusion, operating at a screw speed, particularly of substantially 35 rpm, wherein the composition of filament **A'** is of substantially 92% w/w HPMC and 8% w/w Ketoprofen, and the composition of filament **B'** is of substantially 95% w/w HPMC and 5% w/w polyethylene glycol PEG 400, wherein the extrusion process for both filaments is conducted in the range of 172-174°C, herewith increasing the flow of the molten polymer.

13. Method for producing a multi-layered buccal dosage form according to any one of the claims 9 to 12, wherein the dosage forms are films, **characterized in that** the formation of the drug-loaded compartment (1), the backing layer (2) and the identifiers (3) are solely fabricated in a one-step process expressed as one "build cycle", by 3D printing.

14. Method according to any of the claims 11 to 13, **characterized in that** the produced filaments A', B' are utilized for the fabrication of buccal films with Braille characters in a three-dimensional 3D printing process, to manufacture the dosage forms, based on the designed digital stl templates, wherein the FFF process is performed using a specified printer at an extrusion temperature of 200°C, to generate a smooth flow of the molten polymeric filament from the nozzle of the device, and a build platform temperature of 90°C, thereby increasing adhesion of the object on the platform during the build cycle, wherein the printing speed is set to 30 mm/s, by means whereof the Braille dots (3) are accurately reproduced onto the free surface (21) of the second layer (2), wherein the process parameters are selected in function of the specific material used, depending thereof.

15. Device for carrying out a method as defined according to any one of the claims 8 to 13 resp., **characterized in that** said device comprises a heating chamber and a single-screw hot melt extruder, operating at a certain screw speed,particularly of substantially 35 rpm, by means whereof the filaments A' and B' of HPMC are produced with different compositions, particularly wherein the composition of filament A' is of substantially 92% w/w HPMC and 8% w/w Ketoprofen, and the composition of filament B' is of substantially 95% w/w HPMC and 5% w/w polyethylene glycol PEG 400, wherein the extrusion process for both filaments is conducted in the range of 172-174°C, herewith increasing the flow of the molten polymer in the heating-chamber of said device, wherein said device is further equipped with a nozzle of a diameter particularly of substantially 1,6 mm, thereby yielding filaments with a target diameter of 1,75 ± 0,05 mm.

## Patentansprüche

1. Bukkale Dosierungsform, die zur oralen Verabreichung einer pharmazeutischen Wirkstoffformulierung an sehbehinderte Benutzer gedacht ist, welche mehrschichtig ist,
wobei sie eine erste Schicht (1), die aus einem arzneimittelbeladenen Folienabschnitt besteht, und eine zweite Schicht (2), die als ein Substrat für die Ausbildung und als eine isolierende Abschirmung dienen soll, umfasst, welche zweite Schicht als eine wirksame Barriere dient, welche die Arzneimittelfreisetzung hin zur Mundhöhle begrenzt oder die einseitig gerichtete Freisetzung des Arzneimittels unterstützt und eine Trägerschicht für die Ausbildung von Braille-Identifikatoren (3) bildet, auf welcher die erste Schicht (1) angeordnet und auf einer ersten Seite (21) der zweiten Schicht (2) daran angebracht ist,
wobei die zweite Schicht (2) ferner einen Satz dedizierter Identifikatoren (3) umfasst, die auf der zweiten Schicht (2) der Dosierungsform (10) angeordnet sind, welche die Beladung darstellen,
wobei die zweite Schicht (2) ein Substrat für die Identifikatoren (3) in der Dosierungsform (10) auf der Seite (22) davon (2) bildet, welche ihrer Fixierungsseite (21) entgegengesetzt ist,
wobei die Identifikatoren (3) die Dosierungsstärke der Formulierung angeben,
wobei die Identifikatoren (3) in vorbestimmte Identifikatorenmuster (31) gruppiert sind, die einer spezifischen Beladung entsprechen,
wobei die Identifikatoren (3) von dem sehbehinderten Benutzer ohne Weiteres durch einen taktilen Kontakt damit wahrnehmbar sind, und
wobei die Identifikatoren (3) durch Braille-codierte Zeichen ausgebildet werden, welche auf der zweiten Schicht (2) angebracht sind und welche von der zweiten Schicht (2) hervorstehen, wodurch sie spezifische Braille-codierte Muster (31) bilden, wobei jedes solche spezifische Braille-codierte Muster (31) individuell eine spezifische Beladung für den sehbehinderten Benutzer darstellt.

2. Bukkale Dosierungsform nach Anspruch 1, **dadurch gekennzeichnet, dass** die erste Schicht (1) mit dem arzneimittelbeladenen Abschnitt eine Vielzahl von Dosierungsstärken des pharmazeutischen Wirkstoffs API umfasst, wobei API (*Active Pharmaceutical Ingredient*) für einen aktiven pharmazeutischen Inhaltsstoff steht, und dass die zweite Schicht (2) arzneimittelfrei ist, wodurch sie jeweils eine aktive Schicht und eine passive Trägerschicht darstellen, wobei die Dosierungsform somit eine aktive Schicht und eine passive Schicht mit voneinander unterschiedlicher, jedoch komplementärer Funktionalität beinhaltet.

3. Bukkale Dosierungsform nach Anspruch 1 oder 2, **dadurch gekennzeichnet, dass** das vorbestimmte Identifikatorenmuster (31) rechtwinklig mit variierenden Abmessungen ist, mit dessen Hilfe Dosierungsformen mit unterschiedlichen Dosierungen des aktiven pharmazeutischen Inhaltsstoffes API gekennzeichnet werden.

4. Bukkale Dosierungsform nach einem der Ansprüche 1 bis 3, **dadurch gekennzeichnet, dass** die Fläche des mit Identifikatoren versehenen Abschnitts der zweiten Schicht (2) mit den Abmessungen des arzneimittelbeladenen Abschnitts der ersten Schicht (1) übereinstimmt.

5. Bukkale Dosierungsform nach einem der Ansprüche 1 bis 4, **dadurch gekennzeichnet, dass** die isolierende Abschirmung die Arzneimittelfreisetzung hin zur Mundhöhle eines sehbehinderten Benutzer begrenzt, indem sie den arzneimittelbeladenen Abschnitt der ersten Schicht (1) vor Speichelfluiden des Benutzers schützt, wodurch ein übermäßiges Verschlucken des freigesetzten Arzneimittels durch den Benutzer verhindert wird, wobei die zweite Schicht (2) eine einseitig gerichtete Freisetzung des Arzneimittels hin zum Wangenepithel des sehbehinderten Benutzers verbessert, wobei insbesondere die Dicke der zweiten Schicht 0,1 mm beträgt.

6. Bukkale Dosierungsform nach einem der Ansprüche 1 bis 5, **dadurch gekennzeichnet, dass** die Identifikatoren (3) eine im Wesentlichen zylindrische Form aufweisen und im Wesentlichen orthogonal von der freien Oberfläche (22) der zweiten Schicht (2) als darauf befindliche Braille-Zeichen (3) hervorstehen, welche sich in axialer Richtung entlang einer entsprechenden vertikalen Achse (1) parallel zueinander erstrecken.

7. Bukkale Dosierungsform nach einem der Ansprüche 1 bis 6, **dadurch gekennzeichnet, dass** die Braille-Zeichen einen Punktdurchmesser (32) von im Wesentlichen 1,6 mm, eine horizontale und vertikale Punktbeabstandung (35) bzw. (36) von im Wesentlichen 2,5 mm, eine horizontale Zeichenbeabstandung von im Wesentlichen 6,0 mm und eine vertikale Zeilenbeabstandung von im Wesentlichen 10,0 mm aufweisen, wobei die Punkthöhe (37) im Wesentlichen 0,2 mm beträgt.

8. Verfahren zur Herstellung einer mehrschichtigen bukkalen Dosierungsform wie in einem der Ansprüche 1 bis 7 definiert, **dadurch gekennzeichnet, dass** die Identifikatoren (3) in Übereinstimmung mit der Beladung gemäß einem vorbestimmten Funktionsschema, insbesondere basierend auf einer Proportionalitätsbeziehung zwischen dem Identifikator (3) und der Beladung, ausgebildet werden,
wobei die arzneimittelbeladene Folie (1) ausgebildet wird und diese (1) an der zweiten Schicht (2) angebracht wird, wobei die Identifikatoren (3) als Braille-codierte Zeichen ausgebildet werden, welche eine taktile Identifizierung einer Zieldosis des pharmazeutischen Wirkstoffs durch einen sehbehinderten Benutzer gestatten, und wobei sich das Braille-Format aus der Zieldosisstärke des Wirkstoffs ergibt.

9. Verfahren nach Anspruch 8,
**dadurch gekennzeichnet, dass** es mindestens zwei Phasen umfasst, die Folgendes beinhalten:
- einen ersten Schritt **a,** der aus dem Erzeugen eines Ausgangsmaterials durch Heißschmelzextrusion besteht, und
- einen zweiten Schritt **b,** der aus dem Ausbilden einer Folie als die zweite Schicht (2) einschließlich des Aufbringens von Braille-Zeichen (3) darauf (2) durch 3D-Druck mittels eines Schmelzschichtverfahrens (FFF - *Fused Filament Fabrication*) besteht, wodurch insbesondere eine integrierte Struktur bereitgestellt wird.

10. Verfahren nach dem vorhergehenden Anspruch 9,
**dadurch gekennzeichnet, dass** die Formulierung aus einer festen Kombination des arzneimittelbeladenen Abschnitts als aktive erste Schicht (1), welche unterschiedliche Dosierungsstärken des erforderlichen pharmazeutischen Wirkstoffs API aufweist, mit Aufbringung auf den arzneimittelfreien Abschnitt, welcher die Eigenschaften der Trägerschicht (2) bereitstellt, erzeugt wird, wobei während der gleichen einzelnen Phase des Aufbauzyklus des FFF-Prozesses die Braille-Zeichen (3) auf der Trägerschicht (2) ausgebildet werden, wobei die Braille-Zeichen (3) dem Benutzer ohne Weiteres durch einfachen taktilen Kontakt die Dosierungsstärke der Formulierung anzeigen, wobei diese (3) ferner in spezifische Braille-Muster (31) gruppiert sind, welche das Objekt kennzeichnen, indem sie spezifische Eigenschaften des arzneimittelbeladenen Abschnitts der ersten Schicht (1) angeben, wie z. B. eine Arzneimitteldosis, wobei die Arzneimitteldosis in spezifische Abmessungen des Objekts umgewandelt wird, um so die Zielstärke des Wirkstoffs zu erhalten, wozu ein zusätzlicher Herstellungsprozess durchgeführt wird, wobei ein schichtweiser Materialauftrag auf der Trägerschicht (2) erfolgt, was darin resultiert, dass die Identifikatoren (3) durch den schichtweisen Materialauftrag auf die Trägerschicht (2) ausgebildet werden, für eine haptische Erfassung der darin (3) enthaltenen erforderlichen Informationen durch den jeweiligen Benutzer, der sehbehindert ist.

11. Verfahren nach Anspruch 9 oder 10,
**dadurch gekennzeichnet, dass** arzneimittelfreie Filamente **B'** und arzneimittelbeladene Filamente **A'** aus Hydroxypropylmethylcellulose HPMC durch das Ausführen der folgenden Schritte in der vorgeschriebenen Reihenfolge hergestellt werden:
- der Heißschmelzextrusion-Prozessschritt **a,** wobei die erste Phase aus der Heißschmelzextrusion von Filamenten mit spezifischen Parametern besteht, wodurch die Filamente hergestellt werden, wobei die Filamente das Ausgangsmaterial oder Rohmaterialien für die Realisierung des FFF-Prozessschrittes **b** bilden, und
- eine anschließende Phase, welche die Fertigungsprozedur definiert, die aus dem Design von Mundschleimhautfolien mit Braille-Zeichen (3) besteht.

12. Verfahren nach Anspruch 11,
**dadurch gekennzeichnet, dass** die Filamente **A'** und **B'** aus HPMC in unterschiedlichen Zusammensetzungen unter Verwendung einer Einschnecken-Heißschmelzextrusion, die insbesondere mit einer Schneckendrehzahl von im Wesentlichen 35 U/min arbeitet, hergestellt werden, wobei die Zusammensetzung von Filament **A'** im Wesentlichen aus 92 Gew.-% HPMC und 8 Gew.-% Ketoprofen besteht und die Zusammensetzung von Filament **B'** im Wesentlichen aus 95 Gew.-% HPMC und 5 Gew.-% Polyethylenglykol PEG 400 besteht, wobei der Extrusionsprozess für beide Filamente im Bereich von 172-174 °C durchgeführt wird, wodurch der Fluss des geschmolzenen Polymers erhöht wird.

13. Verfahren zur Herstellung einer mehrschichtigen bukkalen Dosierungsform nach einem der Ansprüche 9 bis 12,
wobei die Dosierungsformen Folien sind, **dadurch gekennzeichnet, dass** der arzneimittelbeladene Abschnitt (1), die Trägerschicht (2) und die Identifikatoren (3) einzig in einem Ein-Schritt-Prozess, ausgedrückt als ein "Aufbauzyklus", durch 3D-Druck gefertigt werden.

14. Verfahren nach einem der Ansprüche 11 bis 13,
**dadurch gekennzeichnet, dass** die hergestellten Filamente **A', B'** für die Fertigung von Mundschleimhautfolien mit Braille-Zeichen in einem dreidimensionalen 3D-Druckprozess zur Herstellung der Dosierungsformen basierend auf den erstellten digitalen STL-Vorlagen genutzt werden, wobei der FFF-Prozess unter Verwendung eines spezifizierten Druckers bei einer Extrusionstemperatur von 200 °C, um einen gleichmäßigen Fluss des geschmolzenen Polymerfilaments aus der Düse der Vorrichtung zu erzeugen, und einer Temperatur der Aufbauplattform von 90 °C, wodurch eine Anhaftung des Objekts auf der Plattform während des Aufbauzyklus erhöht wird, durchgeführt wird, wobei die Druckgeschwindigkeit auf 30 mm/s festgelegt ist, wodurch die Braille-Punkte (3) auf der freien Oberfläche (21) der zweiten Schicht (2) genau wiedergegeben werden, wobei die Prozessparameter in Abhängigkeit vom verwendeten spezifischen Material ausgewählt werden.

15. Vorrichtung zur Ausführung eines Verfahrens wie entsprechend nach einem der Ansprüche 8 bis 13 definiert, **dadurch gekennzeichnet, dass** die Vorrichtung eine Heizkammer und einen Einschnecken-Heißschmelzextruder umfasst, der mit einer bestimmten Schneckendrehzahl, insbesondere im Wesentlichen 35 U/min, arbeitet, wodurch die Filamente **A'** und **B'** aus HPMC in unterschiedlichen Zusammensetzungen hergestellt werden, wobei insbesondere die Zusammensetzung von Filament **A'** im Wesentlichen aus 92 Gew.-% HPMC und 8 Gew.-% Ketoprofen besteht und die Zusammensetzung von Filament **B'** im Wesentlichen aus 95 Gew. -% HPMC und 5 Gew.-% Polyethylenglykol PEG 400 besteht, wobei der Extrusionsprozess für beide Filamente im Bereich von 172-174 °C durchgeführt wird, wodurch der Fluss des geschmolzenen Polymers in der Heizkammer der Vorrichtung erhöht wird, wobei die Vorrichtung ferner mit einer Düse mit einem Durchmesser von im Wesentlichen 1,6 mm ausgestattet ist, wodurch Filamente mit einem Zieldurchmesser von 1,75 ± 0,05 mm entstehen.

## Revendications

1. Forme posologique buccale destinée à l'administration orale d'une formulation de composés pharmaceutiques actifs à des utilisateurs malvoyants, qui est multicouche, comprenant une première couche (1) constituée d'un compartiment pelliculaire chargé de médicament et une seconde couche (2) devant agir comme substrat pour la formation et comme écran isolant servant de barrière effective, qui limite la libération du médicament vers la cavité buccale ou favorise la libération unidirectionnelle du médicament et forme une couche de support pour la formation d'identifiants Braille (3), sur laquelle ladite première couche (1) est disposée et fixée à celle-ci sur un premier côté (21) de ladite seconde couche (2),
ladite seconde couche (2) comprenant en outre un ensemble d'identifiants dédiés (3) qui sont disposés sur ladite seconde couche (2) de ladite forme posologique (10) représentant la charge,
ladite seconde couche (2) constituant un substrat pour lesdits identifiants (3) dans ladite forme posologique (10) sur le côté (22) de celle-ci (2) qui est opposé à son côté de fixation (21),
dans lequel lesdits identifiants (3) indiquent l'intensité de dosage de ladite formulation,
dans lequel lesdits identifiants (3) sont regroupés en motifs prédéterminés d'identifiants (31) correspondant à une charge spécifique,
lesdits identifiants (3) étant perceptibles par ledit utilisateur malvoyant simplement par un contact tactile avec ceux-ci, et
lesdits identifiants (3) étant formés par des caractères codés en Braille, qui sont supportés sur ladite seconde couche (2) et qui font saillie à partir de ladite seconde couche (2), formant ainsi des motifs codés en Braille spécifiques (31), chaque motif codé en Braille spécifique (31) représentant une charge spécifique individuellement pour ledit utilisateur malvoyant.

2. Forme posologique buccale selon la revendication 1, **caractérisée en ce que** ladite première couche (1) avec ledit compartiment chargé de médicament comprend une variété d'intensités de dosages dudit composé pharmaceutique actif API, API désignant un ingrédient pharmaceutique actif, et **en ce que** ladite seconde couche (2) ne contient pas de médicament, constituant ainsi respectivement une couche active et une couche de support passive, ladite forme posologique comprenant ainsi une couche active et une couche passive ayant une fonctionnalité mutuellement différente mais complémentaire.

3. Forme posologique buccale selon la revendication 1 ou 2, **caractérisée en ce que** ledit motif d'identifiants prédéterminé (31) est rectangulaire avec des dimensions variables, au moyen de quoi les formes posologiques ont des doses différentes dudit ingrédient pharmaceutique actif API.

4. Forme posologique buccale selon l'une quelconque des revendications 1 à 3, **caractérisée en ce que** la surface dudit compartiment supportant les identifiants de ladite seconde couche (2) est conforme aux dimensions du compartiment chargé en médicament de ladite première couche (1).

5. Forme posologique buccale selon l'une quelconque des revendications 1 à 4, **caractérisé en ce que** ledit écran isolant limite la libération de médicament vers la cavité buccale d'un utilisateur malvoyant, en protégeant le compartiment chargé de médicament de ladite première couche (1) de fluides salivaires dudit utilisateur, empêchant ainsi une déglutition excessive par ledit utilisateur du médicament libéré, la seconde couche (2) favorisant une libération unidirectionnelle du médicament vers l'épithélium buccal dudit utilisateur malvoyant, en particulier dans laquelle l'épaisseur de ladite seconde couche est de 0,1 mm.

6. Forme posologique buccale selon l'une quelconque des revendications 1 à 5, **caractérisée en ce que** lesdits identifiants (3) ont une forme sensiblement cylindrique faisant saillie pratiquement orthogonalement par rapport à la surface libre (22) de la deuxième couche (2). sous forme de caractères Braille (3) situés sur celle-ci s'étendant axialement le long d'un axe vertical respectif (I) mutuellement parallèle.

7. Forme posologique buccale selon l'une quelconque des revendications 1 à 6, **caractérisée en ce que** lesdits caractères Braille ont un diamètre de point (32) de pratiquement 1,6 mm, un espacement de point horizontal et vertical (33, resp. 34) de pratiquement 2,5 mm, un espacement de caractère horizontal (35) de pratiquement 6,0 mm, un espacement de ligne vertical (36) de pratiquement 10,0 mm, la hauteur du point (37) étant de pratiquement 0,2 mm.

8. Procédé de production d'une forme posologique buccale multicouche telle que définie dans l'une quelconque des revendications 1 à 7, **caractérisé en ce que** lesdits identifiants (3) sont formés en fonction de la charge selon un schéma fonctionnel prédéterminé, notamment basé sur une relation de proportionnalité entre l'identifiant (3) et la charge,
dans lequel ledit film chargé de médicament (1) est formé et il (1) est fixé à ladite seconde couche (2), dans lequel lesdits identifiants (3) sont formés sous forme de caractères codés en Braille permettant une identification tactile d'une dose cible du composé pharmaceutique actif par un utilisateur malvoyant et dans lequel le format Braille est obtenu à partir de la concentration de dose cible du composé actif.

9. Procédé selon la revendication 8, **caractérisé en ce qu'**il comprend au moins deux phases comprenant
- une première étape a consistant à générer de la matière première par extrusion à chaud et
- une deuxième étape b consistant à former un film buccal comme deuxième couche (2) en construisant des caractères Braille (3) dessus (2) par impression 3D en fabriquant des filaments fondus FFF, en particulier fournissant ainsi notamment une structure intégrée.

10. Procédé selon la revendication précédente 9, **caractérisé en ce que** la formulation est générée à partir d'une combinaison fixe dudit compartiment chargé de médicament en tant que première couche active (1) présentant différentes concentrations de dosage du composé pharmaceutique actif API requis avec superposition sur ledit compartiment sans médicament fournissant les propriétés de ladite couche de support (2), dans laquelle, au cours de la même phase unique du cycle de construction dudit processus FFF, lesdits caractères Braille (3) sont formés sur la couche de support (2), dans laquelle lesdits caractères Braille (3) indiquent directement à l'utilisateur la concentration de dosage de la formulation par simple contact tactile, **en ce qu'**ils (3) sont en outre regroupés ensemble dans des motifs Braille spécifiques (31), qui caractérisent l'objet en indiquant des propriétés spécifiques dudit compartiment chargé de médicament de ladite première couche (1), telles que la dose de médicament, ladite dose de médicament étant transformée en dimensions spécifiques dudit objet de manière à atteindre la concentration cible du composé actif, pour lequel un processus de fabrication supplémentaire est effectué dans lequel un dépôt de matériau couche-sur-couche est effectué sur la couche de support (2) résultant **en ce que** les identifiants (3) sont formés par ledit dépôt de matériau couche-sur-couche sur la couche de support (2), pour une appréhension haptique des informations requises contenues dans celles-ci (3) par l'utilisateur concerné qui est malvoyant.

11. Procédé selon la revendication 9 ou 10, **caractérisé en ce que** les filaments B' ne contenant pas de médicament et les filaments A' chargés de médicament d'hydroxypropyl-méthylcellulose HPMC, sont produits en effectuant les étapes suivantes dans l'ordre prescrit :
- l'étape **a** du procédé d'extrusion à chaud, dans laquelle la première phase consiste en ladite extrusion à chaud de filaments avec des paramètres spécifiques préparant lesdits filaments, dans laquelle lesdits filaments constituent la matière première ou les matières premières pour la réalisation de l'étape **b** dudit procédé FFF, et
- une phase ultérieure définissant le processus de fabrication, consistant en la conception de films buccaux avec caractères Braille (3).

12. Procédé selon la revendication 11, **caractérisé en ce que** les filaments **A'** et **B'** de HPMC sont produits avec des compositions différentes en utilisant une extrusion à chaud à vis unique, fonctionnant à une vitesse de vis, en particulier de sensiblement 35 tr/min, dans laquelle la composition du filament **A'** est de sensiblement 92 % p/p de HPMC et 8 % p/p de kétoprofène, et la composition du filament **B'** est de sensiblement 95 % p/p de HPMC et 5 % p/p de polyéthylène glycol PEG 400, dans laquelle le processus d'extrusion pour les deux filaments est réalisé dans la plage de 172 à 174°C, augmentant ainsi le flux du polymère fondu.

13. Procédé de production d'une forme posologique buccale multicouche selon l'une quelconque des revendications 9 à 12, dans lequel les formes posologiques sont des pellicules, **caractérisé en ce que** la formation du compartiment chargé en médicament (1), de la couche de support (2) et des identifiants (3) sont fabriqués uniquement dans un processus en une seule étape exprimé comme un « cycle de construction », par impression 3D.

14. Procédé selon l'une quelconque des revendications 11 à 13, **caractérisé en ce que** le les filaments A', B' produits sont utilisés pour la fabrication de pellicules buccales avec des caractères Braille dans un processus d'impression 3D tridimensionnel, pour fabriquer les formes posologiques, sur la base des modèles stl numériques conçus, le processus FFF étant effectué à l'aide d'une imprimante spécifiée à une température d'extrusion de 200°C, pour générer un écoulement régulier du filament polymère fondu à partir de la buse du dispositif, et une température de plate-forme de construction de 90°C, augmentant ainsi l'adhérence de l'objet sur la plate-forme pendant le cycle de construction, la vitesse d'impression étant réglée à 30 mm/s, au moyen de quoi les points Braille (3) sont reproduits avec précision sur la surface libre (21) de la seconde couche (2), les paramètres du processus étant sélectionnés en fonction du matériau spécifique utilisé, en fonction de celui-ci.

15. Dispositif pour la mise en oeuvre d'un procédé tel que défini selon l'une quelconque des revendications 8 à 13, **caractérisé en ce que** ledit dispositif comprend une chambre de chauffage et une extrudeuse de matière fondue à vis unique, fonctionnant à une certaine vitesse de vis, en particulier de sensiblement 35 tr/min, au moyen de laquelle les filaments **A'** et **B'** de HPMC sont produits avec des compositions différentes, en particulier dans laquelle la composition du filament **A'** est de sensiblement 92 % p/p de HPMC et 8 % p/p de kétoprofène, et la composition du filament **B'** est de sensiblement 95 % p/p de HPMC et 5 % p/p de polyéthylène glycol PEG 400, dans lequel le processus d'extrusion pour les deux filaments est conduit dans la plage de 172 à 174°C, augmentant ainsi le flux du polymère fondu dans la chambre de chauffage dudit dispositif, dans lequel ledit dispositif est en outre équipé d'une buse d'un diamètre notamment de sensiblement 1,6 mm, ce qui permet d'obtenir des filaments avec un diamètre cible de 1,75 ± 0,05 mm.
